# EUROPEAN PATENT APPLICATION

(11) **EP 4 835 770 A1**
(43) Date of publication of application: **04.11.2026**
(21) Application number: 25173781.3
(22) Date of filing: 30.04.2025
(51) Int. Cl.: G06Q 10/10, G06Q 10/109, G16H 40/20

(54) **METHOD AND SYSTEM FOR SERVICE TOKEN MANAGEMENT AND AUTOMATION FLOW**

(71) Applicant: Mitel Networks Corporation, Ottawa, Ontario K2K 3K1 (CA)
(72) Inventor: Brochonski, Michael, 80730-000 Curitiba (BR); Braganza, Jonathan, Neepan, K2E 6P2 (CA); Naidoo, Logendra, Ottawa, K1S 0W8 (CA)
(74) Representative: Schaafhausen, Anne

(57) **Abstract**

The invention relates to a method and system for service token management and automation flow. The method and system specifically relate to digital wallets that can be used to manage and track appointments (e.g. medical appointments), follow-ups and related workflows to simplify a process and reduce the need to juggle between multiple systems. The familiarity and convenience of digital wallets could make it easier to handle everything from scheduling appointments to accessing records to confirming task completion through a single, accessible platform.

## Description

The present invention relates to a method and system for service token management and automation flow. The method and system specifically relate to digital wallets that can be used to manage and track appointments (e.g. medical appointments), follow-ups and related workflows to simplify a process and reduce the need to juggle between multiple systems. The familiarity and convenience of digital wallets could make it easier to handle everything from scheduling appointments to accessing records to confirming task completion through a single, accessible platform.

Managing appointments like for example medical appointments, examinations, and other service-based processes is often cumbersome and inefficient. Users frequently face difficulties in keeping track of appointments, scheduling follow-ups, and ensuring that each step in the process is completed in the correct order. The complexity increases with multiple appointments, requiring users to search for the right information, manually enter details, and remember the necessary steps to complete each task. This manual process not only leads to missed or delayed appointments but also adds unnecessary stress for individuals who rely on these services regularly.

Scheduling and managing medical appointments, exams, and related tasks can be a frustrating and inefficient process. Users often face the challenge of manually tracking appointments, scheduling follow-ups, and ensuring each step is completed in the right order. This can involve checking emails, using calendars, and juggling information across different apps or platforms. Keeping up with these tasks, particularly when multiple appointments or tests are involved, is prone to errors and missed steps, resulting in delays and complications.

In the past, users relied on dedicated healthcare apps or calendar tools to manage appointments. While these systems allow scheduling and reminders, they still require users to handle follow-ups and track whether critical tasks - such as lab results or prescriptions - have been completed. This typically means accessing different applications and manually keeping track of records and appointments, which introduces the risk of oversight.

Other solutions, such as appointment reminders or QR codes for check-ins, address some of these issues but still fall short of providing a cohesive system. These approaches don't integrate well with the tools users already use regularly, such as digital wallets, making the process more fragmented and cumbersome.

Currently, there is no solution that manage the entire lifecycle of a service task or healthcare tasks.

Therefore, the present invention is based on the object to provide a method and a corresponding system for service token management and automation flow.

This object is solved by a method having the features according to claim 1 and a system having the features of claim 11. Preferred embodiments of the invention are defined in the respective dependent claims.

According to the invention, a method for service token management and automation flow is provided, comprising the steps of:
- defining, by a service provider, an event or a service process with a sequence of steps;
- generating, by a backend service module, a digital service token which comprises the defined event or service process;
- distributing, by the backend service module, the digital service token to a user device;
- interacting, by a user of the user device, with the digital service token by means of a frontend interface of a designated platform;
- indicating, by the user over the frontend interface of the designated platform and/or by the backend service module, that a first step of the sequence of steps is completed;
- verifying, by the designated platform and/or backend service module, that the first step in the sequence of steps of the digital service token has been completed;
- updating, by the designated platform and/or the backend service module, the digital service token in real-time;
- triggering, by the digital service token, the designated platform and/or the backend service module to automatically advance to the next step in the sequence of steps of the event or the service process;
- repeating, by the user, by the designated platform and/or by the backend service module, the steps of the method of indicating, verifying and updating completed steps of the sequence of steps of the digital service token and of triggering to automatically advance to the next step in the sequence of steps until all the steps of the sequence of steps, of the event or the service process of the digital service token are finished.

In the context of the present invention, the designated platform can, for example, refer to a digital wallet system (e.g. Apple Wallet, Google Wallet) that serves as a primary user interface for interacting with the digital service token. In the case of a digital wallet system, this designated platform is transformed from a simple payment and ticket storage system into a privacy-focused workflow management function. It becomes the centralized location where users access, view, and interact with their digital service tokens, enabling them to track appointments, trigger next steps, and manage the entire process lifecycle.

In the context of the present invention, the backend service module is responsible but not limited for one or more of the following example actions digital service token generation, distribution, verification, and orchestration of the multi-step processes. It integrates with scheduling systems and service providers' databases to ensure all details remain current and actionable. This backend service handles the logic for validating task completion, managing the sequential progression of steps, and updating digital service token status. It maintains connections with external systems (like healthcare providers' systems) while preserving data privacy through encryption and selective information sharing based on context and user permissions.

In the context of the present invention, the digital service token is a core digital construct that encapsulates all information related to a service process. It can comprise essential details about appointments, tasks, deadlines, participant information, time-based triggers, and the sequence of required actions. This "smart container" can actively manage process workflows, tracks completion status, stores history of past actions for traceability, and selectively shares data with appropriate stakeholders. The digital service token can be dynamically updated to reflect current status and only presents relevant next steps based on progress.

In the context of the present invention the user device is the physical device (for example smartphone, tablet, computer) where the designated platform (e.g. digital wallet application), or parts whereof, is installed and through which the user interacts with the digital service token. The device can thereby receive notifications about token updates and provide secure access to the designated platform environment.

In the context of the present invention, the frontend interface is a user-facing component of the designated platform where digital service tokens are displayed, and interactions occur. This interface presents the digital service token's visual representation. This can comprise relevant details, status updates, and action buttons that trigger workflows. It handles user inputs for completing tasks or confirming actions and displays only the currently relevant information to reduce complexity.

In the context of the present invention, a service provider is an entity (e.g. hospital, clinic, laboratory, education institution, etc.) that defines the service process with its required sequence of steps. The provider establishes the workflow requirements, specifies task dependencies (e.g., blood test before MRI), and determines what information needs to be included in the token. They may receive verification of task completion through the backend service.

In the context of this invention, the user is an individual who receives and interacts with the digital service token. The user adds the digital service token to his designated platform, views details about his tasks and appointments, and triggers actions to advance through the process. The user maintains control over his personal data through privacy permissions while benefiting from the automated workflow management. This architecture ensures HIPAA compliance and addresses privacy concerns through encryption and contextual data sharing, transforming designated platforms (e.g. digital wallets) from static storage into active process managers that reduce manual effort and ensure sequential task completion.

According to the invention, the digital service token can be delivered to the user via an existing platform, such as email, a messaging service, or direct into a digital wallet. Optionally, the user receives a notification or prompt to accept the digital service token. The system verifies by means of the backend service module and/or the designated platform (e.g. a digital wallet) that each step or task is completed before proceeding to the next step, thus ensuring accuracy and preventing incomplete processes from moving forward. This can comprise confirmation from external systems that medical tests are completed or payments are confirmed. The digital service token is updated in real-time as steps or tasks are completed or modified, reflecting the current status of the service process. The user is presented only with the next relevant step or action based on their progress. When the user completes a step, task or action, the token triggers the system to automatically advance to the next step in the sequence of steps. The steps of the process are completed in order, ensuring no steps are missed or overlooked. The process of step completion is repeated, and the digital service token is continuously updated until all steps of the sequence of steps defined by the service token are completed. For the purposes of the invention, a user device is any type of device with which a user can gain access to the content of the digital service token and can also interact with it. For example, this can be a smartphone, a smartwatch, a computer or any other suitable device.

According to a preferred embodiment, the first step and/or any next step of the sequence of steps is one or more of a task, an action, an appointment, an exam, a deadline, a workflow, a follow-up, a progress tracking, a trigger, an alarm, a prompt or a notification.

Optionally, this comprises steps or tasks that may span past, present, and future appointments or actions. As example, this can be broken down to an incident with different steps as follows. There is a main incident which comprises a series of events/steps, which can differ in scope. Each event is a separate requirement which must be completed before the incident (or sequence of steps) is marked as complete. Each individual event may or may not be ordered, depending on the specific case. An example of this could be a doctor examining a patient for a specific illness, and the doctor is trying to determine what the cause is. Therefore, the doctor has ordered multiple tests to be completed, in any order before the tests will be analyzed for results and finally to determine the outcome. The following tests have been requested: blood work, x-ray, MRI, cat scan. These tests/steps must all be completed before the doctor is notified and the results will be examined.

According to another preferred embodiment, the digital service token is one or more of a digital wallet card or service pass, a digital event cart or pass and/or a digital step-by-step service guide. The digital service token encapsulates all required information for the multi-step process, comprising tasks, deadlines, and required actions. The token serves as a singular reference point for tracking the process and triggering relevant workflows.

According to still another preferred embodiment, the frontend interface of the designated platform is one or more of a display, a touch display, an audio system, a text-to-speech, tts, module, a speech-to-text, stt, module, one or more button, rotary encoder or key, or any means by which a user can interact with the designated platform. In the context of the invention, frontend refers to an interface with which a user can interact. An example may be an application in which the user can touch or click on areas of a screen to make inputs.

Further, according to a preferred embodiment, the designated platform is a digital wallet. The user can access the digital service token through the designated platform and view all relevant details about the upcoming steps, tasks, appointments etc. Upon interaction, the designated platform triggers the next required step in the workflow.

According to yet another preferred embodiment, the digital service token comprises a backward traceability to review any part of the event or the service process. The digital service token retains the history of past steps, like tasks and appointments, allowing users and providers to track completed actions and review any part of the process. This can be used to enable auditing and error-checking if needed.

According to yet another preferred embodiment, the method further comprises triggering by the designated platform and/or the backend service module intelligent recommendations to the user by means of an artificial intelligence module. The digital service token can be used to analyze user behavior and past actions to offer intelligent recommendations for future tasks or actions based on patterns or missed steps. For example, in a healthcare setting, it might suggest scheduling an annual check-up if none is recorded in the past year.

According to yet another preferred embodiment, the method further comprises archiving or reactivating, manually by the user or automatically by the designated platform and/or the backend service module, the digital service token for record-keeping or for future use. Once the whole process is finished, the digital service token can either be archived for record-keeping or reactivated for future use.

According to yet another preferred embodiment, the backend service module is or is part of one or more of a computer, a server, an application, computer-programmable product or a software as a service (SaaS).

According to yet another preferred embodiment, the service provider is one or more of a healthcare provider, a governmental authority, an educational institution or university, an insurance provider, a financial provider, transportation provider, logistic provider and/or a network and telecommunication provider.

According to the invention, a system is provided which is configured to perform the method for service token management and automation flow according to any one of the above-mentioned embodiments.

It has also to be noted that aspects of the invention have been described with reference to different subject-matters. In particular, some aspects or embodiments have been described with reference to apparatus type claims whereas other aspects have been described with reference to method type claims. However, a person skilled in the art will gather from the above and the following description that, unless otherwise notified, in addition to any combination between features belonging to one type of subject-matter also any combination between features relating to different types of subject-matters is considered to be disclosed with this text. In particular combinations between features relating to the apparatus type claims and features relating to the method type claims are considered to be disclosed. In addition, features relating to one of the embodiments may be combined with other features of another embodiment, the drawings or the claims, where possible. The invention and embodiments thereof will be described below in further detail in connection with the drawing(s).
- Fig. 1: shows a flowchart of a healthcare digital event card creation and scheduling flow according to an embodiment of the invention.
- Fig. 2: shows a flowchart of a follow-up and medical record management flow according to another embodiment of the invention.

Even if the invention is described in more detail below on the basis of a process located in the field of healthcare, it is not limited to this area but can be applied to all areas in which multi-step processes play a role.

Thus, beyond orchestrating healthcare appointments, this invention can be applied to other service-based processes that require coordination and task management. For instance, it could be used for scheduling and managing tasks such as exam scheduling, job interviews, or booking services like travel or personal care appointments. The participants receive a digital event card with all necessary details and a trigger to complete the next step. The same method could be applied to manage recurring appointments or tasks in various sectors, ensuring users have all the information they need in a convenient and accessible format.

Existing solutions for managing healthcare appointments typically rely on separate scheduling tools, apps, or manual tracking, requiring users to handle tasks across multiple platforms. This often results in unnecessary complexity, as users need to input details, manage follow-ups, and track records manually. These systems rarely integrate with tools that users already have at their disposal, such as digital wallets, leaving gaps in efficiency and ease of use.

This invention leverages the existing capabilities of a designated platform e.g. digital wallets to handle the entire lifecycle of healthcare management, from appointment scheduling to follow-up tasks and record tracking. By using Digital Event Cards within the wallet, the process becomes centralized. Information about appointments, tasks, and test results is tied directly to the wallet, and all related actions - such as scheduling or confirmations - can be triggered from one place.

The core difference lies in automating workflows. Once a Digital Event Card is received, it acts as a step-by-step guide for the user, initiating tasks such as appointment confirmations, exam scheduling, and follow-up reminders, all without the need for manual input or switching between applications. This ensures that necessary steps are completed in sequence, reducing the risk of missed appointments or overlooked tasks. The wallet becomes more than a repository for reminders; it is the control center for all interactions related to healthcare processes.

By embedding dynamic content and triggers into the wallet card, the system displays only relevant next steps after the previous tasks are completed. This reduces user confusion and simplifies the overall process, making it significantly more efficient than current methods which still rely heavily on user input and fragmented systems.

This invention introduces a method for managing service-based processes through the integration of a centralized, automated system, using a digital wallet as the medium. The core concept revolves around creating a digital construct, such as a service pass, that contains essential information related to a series of tasks or events, including appointments, follow-ups, and progress tracking. By consolidating these elements into a single, manageable entity within a digital wallet, users can interact with multiple processes seamlessly, without needing manual input at every stage.

What sets this method apart is its emphasis on automation and real-time task management. The system not only records and tracks key details but also initiates relevant workflows, such as verifying task completion before advancing to the next stage. This eliminates the need for users to interact with multiple platforms or manually update their progress. The method is designed to integrate with existing infrastructures while enhancing productivity by reducing redundant steps and centralizing information in a coherent framework.

By providing real-time updates and task prompts, the system simplifies complex, multi-step processes, ensuring that each stage is completed before the next action is triggered. This reduces the administrative burden on users and offers an efficient method for managing service-based processes, particularly in fields such as healthcare, education, or professional services. The automation ensures that tasks are completed accurately and efficiently, with minimal user intervention.

Additionally, this method can be extended to other domains, such as education, where students can receive digital event cards for class schedules and exam notifications, or professional services where clients receive cards with appointment details and reminders. The versatility of this method makes it applicable to a wide range of scenarios where coordination and ease of access are crucial. The digital wallet format ensures that all actions are consolidated into a familiar platform, minimizing errors and improving efficiency.

The invention transforms digital wallets from simple payment and ticket storage systems into privacy-focused workflow management functions. In other words, the invention transforms the wallet from a payment tool into a task-driven assistant that actively reduces user steps and integrates seamlessly with service-based, multi-step processes. Current digital wallets store static information (payment cards, boarding passes, or basic event tickets). The invention proposes a new self-contained ecosystem that manages complex, multi-step processes while maintaining strict data privacy controls. Thereby, orchestrating sequential tasks across different service providers (hospitals, labs, hotels, venues) while keeping sensitive personal data encrypted and under user control. Today digital wallets lack the ability to handle dependent tasks (e.g., like Patient X requires a blood test before an MRI) or manage identity-dependent access to personal information (i.e. sharing medical history only with relevant healthcare providers). The invention creates a "smart container" to store information and actively manage process workflows, tracks completion status, and selectively share data based on context and user permission. Therefore, the invention can target, for example, HIPAA compliance and address privacy concerns that traditionally limit digital integration, and hospitality, where guest services often remain fragmented across different systems.

This invention outlines a method for managing multi-step processes through the use of a digital construct, which can be deployed within existing platforms such as digital wallets. The digital construct, referred to as a service pass, encompasses all relevant information needed to complete various tasks, including scheduling details, follow-up actions, participant information, and time-based triggers. The method allows users to interact with this construct to initiate, monitor, and complete tasks, with the system automating the progression from one step to the next.

The underlying architecture involves both a backend service responsible for generating the digital service passes and a frontend interface where users can manage their interactions. The backend is integrated with scheduling or task management systems to ensure that all details remain current and actionable. The frontend interface, integrated with platforms like digital wallets, enables users to select their service pass and trigger the necessary workflows, facilitating the completion of each step in a structured sequence.

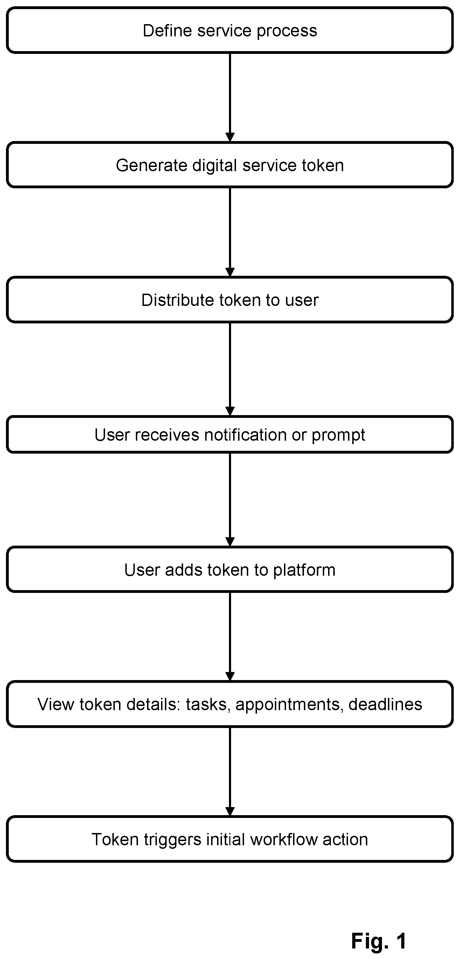

Fig. 1 schematically shows a flowchart of healthcare digital event card creation, distribution and scheduling flow.

The process begins with a service provider or organizer defining the event or service process. The service provider (like a healthcare provider) defines the steps required, such as scheduling an appointment, tests, or follow-ups.

Once defined, the system generates a digital token or a digital service (token) pass. This pass contains all necessary information and includes triggers that automate the next actions in the workflow. This token is then sent to the user who optionally receives a notification (via email, message, or his designated platform, e.g. a digital wallet). The user adds the token to his designated platform, for example a digital wallet (such as Apple Wallet or Google Wallet), where he can view the details, including tasks, deadlines, and future appointments. It is also possible that the token is automatically integrated into the designated platform. The token then can be configured to automatically trigger a first action or step of a sequence of steps, such as scheduling the initial appointment or confirming an upcoming one.

At the appointed time or upon receiving a notification, the user interacts with the pass, which then triggers the system to initiate the corresponding task, such as confirming attendance, scheduling follow-ups, or verifying completion of previous steps.

One key aspect of this invention is the automation of task progression. By interacting with the service pass, users can trigger applications or systems that automatically update the status of their tasks, reducing the potential for errors typically associated with manual tracking.

The system also dynamically updates the pass to reflect the next steps, ensuring that the user is presented only with the tasks relevant to their current progress. This automated process reduces manual input, improves accuracy, and ensures that all tasks are completed in the correct order.

This method is designed to be adaptable across various service-based activities, enabling structured automation for complex, multi-step processes. The service pass model allows for real-time task management, dynamic content delivery, and seamless integration with existing systems, offering a practical solution for industries that require precision in task orchestration, such as healthcare, education, and professional services.

In the following a typical process flow is depicted. However, not all steps of these flows must always be carried out. This flow example shows what is generally possible without limiting the invention. For example, it may also be possible to reorganize, restructure, combine, add or drop one or more steps as the service provider deems appropriate.
1. The service provider defines the event or service process. The provider identifies the multi-step process, which could include tasks, actions, and scheduled appointments. This process includes tasks that may span past, present, and future appointments or actions.
2. The method/system generates a digital service token. The token encapsulates all required information for the multi-step process, including tasks, deadlines, and required actions. The token serves as a singular reference point for tracking the process and triggering relevant workflows.
3. The token is distributed to the user. The token is delivered to the user via an existing platform, such as email, a messaging service, or a digital wallet. The user can receive a notification or prompt to accept the token.
4. The user interacts with the token. The user accesses the token through the designated platform and views all relevant details about their tasks and appointments. Upon interaction, the system triggers the next required step in the workflow.
5. The token updates dynamically. The token is updated in real-time as tasks are completed or modified, reflecting the current status of the process. The user is presented only with the next relevant action based on their progress.
6. The method/system automates task progression. When the user completes a task or action, the token triggers the system to automatically advance to the next step in the sequence. Tasks are completed in order, ensuring no steps are missed or overlooked.
7. The method/system verifies task completion. The system verifies that each task is completed before proceeding, ensuring accuracy and preventing incomplete processes from moving forward. This can include confirmation from external systems, such as medical tests completed or payments confirmed.
8. The token includes backward traceability. The token retains the history of past tasks and appointments, allowing users and providers to track completed actions and review any part of the process. This enables auditing and error-checking if needed.
9. The method/system triggers intelligent recommendations. The token can be used to analyze user behavior and past actions, offering intelligent recommendations for future tasks or actions based on patterns or missed steps. For example, in a healthcare setting, it might suggest scheduling an annual check-up if none is recorded in the past year.
10. The process is repeated and finalized. The token continues to update and manage tasks until all actions are completed. Once the process is finished, the token can either be archived for record-keeping or reactivated for future appointments or actions.

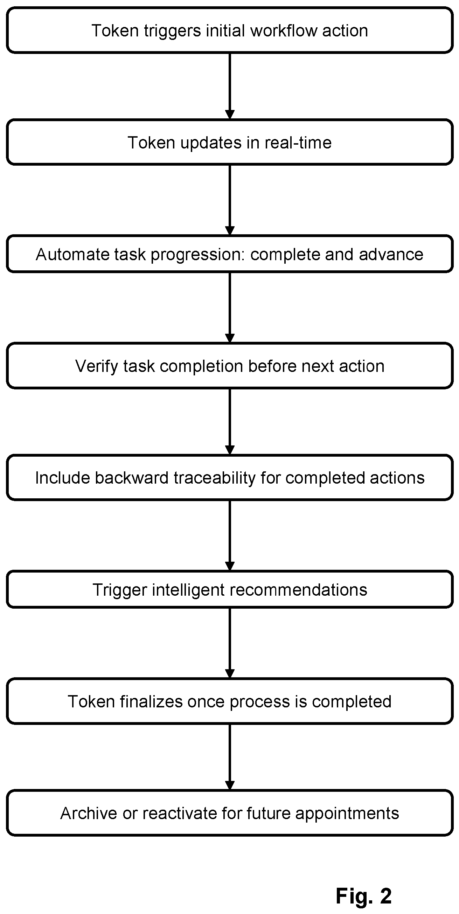

Fig. 2 shows in a flowchart how the token is managed and updated. Once the user interacts with it, the token updates in real-time, reflecting completed tasks and upcoming steps. As each task is completed, the token advances to the next step, such as scheduling a follow-up. The method/system verifies task completion and tracks past actions, like showing finished appointments. It can also provide recommendations, like suggesting the next appointment based on history. When all tasks are complete, the token either finalizes and stores the record or reactivates for future actions. In a wallet, the token handles everything directly, simplifying the process.

It should be noted that the term "comprising" does not exclude other elements or steps and the "a" or "an" does not exclude a plurality. Further, elements described in association with different embodiments may be combined.

It should also be noted that reference signs in the claims shall not be construed as limiting the scope of the claims.

## Claims

1. A method for service token management and automation flow comprising the steps of:
- defining, by a service provider, an event or a service process with a sequence of steps;
- generating, by a backend service module, a digital service token which comprises the defined event or service process;
- distributing, by the backend service module, the digital service token to a user device;
- interacting, by a user of the user device, with the digital service token by means of a frontend interface of a designated platform;
- Indicating, by the user over the frontend interface of the designated platform and/or by the backend service module, that a first step of the sequence of steps is completed;
- verifying, by the designated platform and/or backend service module, that the first step in the sequence of steps of the digital service token has been completed;
- updating, by the designated platform and/or the backend service module, the digital service token in real-time;
- triggering, by the digital service token, the designated platform and/or the backend service module to automatically advance to the next step in the sequence of steps of the event or the service process;
- repeating, by the user, by the designated platform and/or by the backend service module the steps of the method of indicating, verifying and updating completed steps of the sequence of steps of the digital service token and of triggering to automatically advance to the next step in the sequence of steps until all the steps of the sequence of steps of the event or the service process of the digital service token are finished.

2. The method according to claim 1, wherein the first step and/or any next step of the sequence of steps is one or more of a task, an action, an appointment, an exam, a deadline, a workflow, a follow-up, a progress tracking, a trigger, an alarm, a prompt or a notification.

3. The method according to any one of the preceding claims, wherein the digital service token is one or more of a digital wallet card or service pass, a digital event cart or pass and/or a digital step-by-step service guide.

4. The method according to any one of the preceding claims, wherein the frontend interface of the designated platform is one or more of a display, a touch display, an audio system, a text-to-speech, tts, module, a speech-to-text, stt, module, one or more button, rotary encoder or key, or any means by which a user can interact with the designated platform.

5. The method according to any one of the preceding claims, wherein the designated platform is a digital wallet.

6. The method according to any one of the preceding claims, wherein the digital service token comprises a backward traceability to review any part of the event or the service process.

7. The method according to any one of the preceding claims, wherein the method further comprises triggering, by the designated platform and/or the backend service module, intelligent recommendations to the user by means of an artificial intelligence module.

8. The method according to any one of the preceding claims, wherein the method further comprises archiving or reactivating, manually by the user or automatically by the designated platform and/or the backend service module, the digital service token for record keeping or for future use.

9. The method according to any one of the preceding claims, wherein the backend service module is or is part of one or more of a computer, a server, an application, computer-programmable product or a software as a service (SaaS).

10. The method according to any one of the preceding claims, wherein the service provider is one or more of a healthcare provider, a governmental authority, an educational institution or university, an insurance provider, a financial provider, transportation provider, logistic provider and/or a network and telecommunication provider.

11. A system configured to perform the method for service token management and automation flow according to any of the claims 1 to 10.
